(19) Europäisches Patentamt
European Patent Office
Office européen des brevets

(11) **EP 0 893 117 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**08.02.2012 Patentblatt 2012/06**

(51) Int Cl.:
***A61K 8/73*** *(2006.01)* ***A61K 8/81*** *(2006.01)*
***A61Q 5/02*** *(2006.01)* ***A61Q 5/12*** *(2006.01)*

(45) Hinweis auf die Patenterteilung:
**17.11.2004 Patentblatt 2004/47**

(21) Anmeldenummer: **98112651.9**

(22) Anmeldetag: **08.07.1998**

(54) **Verwendung von kationischen Polymeren als Haarkonditionierungsmittel**

Use of cationic copolymers as hair conditioning agents

Utilisation de polymères cationiques comme agents de soin capillaire

(84) Benannte Vertragsstaaten:
**DE ES FR GB IT**

(30) Priorität: **24.07.1997 DE 19731764**

(43) Veröffentlichungstag der Anmeldung:
**27.01.1999 Patentblatt 1999/04**

(73) Patentinhaber: **BASF SE**
**67056 Ludwigshafen (DE)**

(72) Erfinder:
- **Dieing, Reinhold, Dr.**
  **67105 Schifferstadt (DE)**
- **Hössel, Peter, Dr.**
  **67105 Schifferstadt (DE)**
- **Kothrade, Stephan, Dr.**
  **67117 Limburgerhof (DE)**
- **Sanner, Axel, Dr.**
  **67227 Frankenthal (DE)**
- **Zeitz, Katrin, Dr.**
  **67067 Ludwigshafen (DE)**
- **Raubenheimer, Hans-Jürgen**
  **68775 Ketsch (DE)**
- **Schehlmann, Volker, Dr.**
  **67354 Römerberg (DE)**

(56) Entgegenhaltungen:
**EP-A- 0 671 157** **EP-A1- 0 761 206**
**EP-A2- 0 524 434** **WO-A1-94/21224**
**WO-A1-97/35544** **US-A- 4 806 345**

EP 0 893 117 B2

## Beschreibung

Die vorliegende Erfindung betrifft die Verwendung von Polymeren, erhältlich durch radikalisch initiierte Copolymerisation von Monomergemischen aus

(a) 2 bis 70 Gew.-% eines kationischen Monomeren oder quaternisierbaren Monomeren,
wobei als Monomer (a)
N-Vinylimidazol-Derivate der allgemeinen Formel (I),

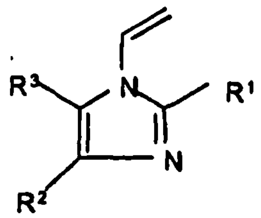

(I)

worin $R^1$ bis $R^3$ für Wasserstoff, C1 - C4 -Alkyl oder Phenyl steht, oder
Diallylamine der allgemeinen Formel (II),

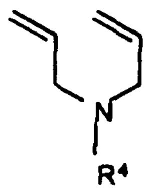

(II)

worin $R^4$ für C1 - C24 -Alkyl steht
oder
3-Methyl-1-vinylimidazoliumchlorid, -methosulfat, Dimethyldiallyammoniumchlorid
verwendet werden,
(b) 22-97,98 Gew.-% N-Vinylpyrrolidon,
(c) 0 bis 40 Gew.-% eines weiteren radikalisch copolymerisierbaren Monomeren und
(d) 0,02 bis 8 Gew.-% eines bi- oder mehrfunktionellen radikalisch copolymerisierbaren Monomeren, und

anschließende Quaternisierung des Polymeren, sofern als Monomeres (a) ein nicht quaternisiertes Monomer eingesetzt wird, als Haarkonditioniermittel in Zubereitungen für die Haarkosmetik, insbesondere in Shampoos.

Kationische Polymere werden als Konditioniermittel in kosmetischen Formulierungen eingesetzt. Sie bewirken in erster Linie eine Verbesserung der Naßkämmbarkeit des Haares. Außerdem verhindern kationische Polymere die elektrostatische Aufladung des Haares.

In Shampoos werden vor allem kationische Zellulose-Derivate (Polyquaternium 10) eingesetzt. Allerdings beobachtet man bei diesen Verbindungen einen build-up Effekt, d. h. das Haar wird bei mehrfacher Anwendung mit dem Conditioner belegt und fühlt sich beschwert an.

Ferner finden Copolymere aus Acrylamid und Dimethyldiallylammoniumchlorid (Polyquaternium 7) Verwendung. Diese haben allerdings den Nachteil hoher Restmonomerengehalte, da Acrylamid und Dimethyldiallylammoniumchlorid ungünstige Copolymerisationsparameter aufweisen.

Aufgabe der vorliegenden Erfindung war es, ein kationisches Konditioniermittel für Shampoos mit verbesserter Wirksamkeit und ohne build-up Effekt zu finden.

Quaternisierte Polymere und ihre Verwendung als Konditioniermittel in Haarpflegeformulierungen sind bekannt.

So wird in EP 246580 die Verwendung von Homo- und Copolymeren von 3-Methyl-1-vinylimidazoliumchloriden u. a. als Haarkonditioniermittel beschrieben. EP 544158 und US 4 859 756 beanspruchen die Verwendung von Homo- und Copolymeren von chloridfreien, quaternisierten N-Vinylimidazolen in kosmetischen Zubereitungen. Aus EP 715843 ist die Verwendung von Copolymeren aus einem quaternierten N-Vinylimidazol, N-Vinylcaprolactam und N-Vinylpyrrolidon sowie optional einem weiteren Comonomer in kosmetischen Zubereitungen bekannt.

In keinem der genannten Patente ist die Verwendung von vernetzten Polymeren beschrieben.

DE 3209224 beschreibt die Herstellung von vernetzten Polymerisaten auf Basis N-Vinylpyrrolidon und (quaternisiertem) N-Vinylimidazol. Diese Polymerisate werden für die Verwendung als Adsorbentien und Ionenaustauscher beansprucht. Sie sind hochvernetzt, wasserunlöslich, wenig quellbar und daher nicht geeignet als Konditioniermittel in kosmetischen Formulierungen.

Aus US 4 058 491 sind vernetzte kationische Hydrogele aus N-Vinylimidazol oder N-Vinylpyrrolidon und einem quaternierten basischen Acrylat sowie weiteren Comonomeren bekannt. Diese Gele werden zur Komplexierung und kontrollierten Freisetzung anionischer Wirksubstanzen vorgeschlagen.

WO 96/26229 beschreibt vernetzte Copolymere aus N-Vinylimidazolen und u. a. quaternisierten N-Vinylimidazolen. Die Polymerisate werden als Zusätze für Waschmittelformulierungen zur Inhibierung der Farbstoffübertragung und als Adsorbentien vorgeschlagen. Sie sind wasserunlöslich und für kosmetische Formulierungen nicht geeignet.

WO 96/37525 beschreibt die Herstellung von vernetzten Copolymeren aus u. a. N-Vinylpyrrolidon und quaternierten Vinylimidazolen in Gegenwart von Polymerisationsreglern und ihre Verwendung insbesondere in Waschmitteln.

DE 4213971 beschreibt Copolymerisate aus einer ungesättigten Carbonsäure, quaternisiertem Vinylimidazol und optional weiteren Monomeren und einem Vernetzer. Die Polymere werden als Verdickungs- und Dispergiermittel vorgeschlagen.

DE 2821239 (US 4348380) beschreibt Copolymere von quaternisierten Diallylammoniumverbindungen in haarkosmetischen Zubereitungen. Die Polymere sind unvernetzt.

DE 3106974 beansprucht ein Haarbehandlungsmittel vom Vorshampooniertyp, das Homo- und Copolymere quaternisierter Diallylammoniumverbindungen enthält. Ein Vernetzer wird nicht erwähnt.

US 5275809, EP 522755, EP 521665 und EP 521666 beanspruchen Copolymere mit Dimethyldiallylammoniumchlorid für die Verwendung in Shampoos. In keiner der vorstehend genannten Schriften ist ein vernetztes Polymer beschrieben.

US 4 806 345 nennt vernetzte kationische Verdicker für kosmetische Formulierungen aus quaterniertem Dimethylaminoethylmethacrylat und Acrylamid.

WO 93/25595 nennt vernetzte kationische Copolymere auf Basis quaternierter Dialkylaminoalkylacrylate oder Dialkylaminoalkylacrylamiden. Als Anwendung wird der Einsatz dieser vernetzten Copolymere als Verdicker in kosmetischen Zubereitungen vorgeschlagen, z. B. in Shampoo-Formulierungen.

EP 0671157 beschreibt Verdicker auf Basis von vernetzten kationischen Polymeren, die aus (Meth)acrylmonomeren, die eine Aminogruppe tragen, aufgebaut sind.

Überraschenderweise wurde gefunden, daß die eingangs definierten vernetzten Polymere eine sehr gute konditionierende Wirkung in Shampoos aufweisen, während die entsprechenden unvernetzten Polymere nur eine geringe Wirksamkeit als Haarkonditioniermittel in Shampoos zeigen.

Die erfindungsgemäße Verwendung betrifft Polymere, die erhältlich sind durch (i) radikalisch initiierte Copolymerisation von Monomergemischen aus

(a) 2 bis 70 Gew.-%, besonders bevorzugt 2 bis 50 Gew.-% eines kationischen Monomeren oder quaternisierbaren Monomeren wie in Anspruch 1 definiert,

(b) 22 bis 97,98 Gew.-%, besonders bevorzugt 45 bis 97,95 Gew.-% N-vinylpyrrolidon,

(c) 0 bis 40 Gew.-% besonders bevorzugt 0 bis 30 Gew.-% eines weiteren radikalisch copolymerisierbaren Monomeren und

(d) 0,02 bis 8 Gew.-%, besonders bevorzugt 0,05 bis 5 Gew.-% eines bi- oder mehrfunktionellen radikalisch copolymerisierbaren Monomeren,

und (ii) anschließende Quaternisierung des Polymeren, sofern als Monomeres (a) ein nicht quaternisiertes Monomer eingesetzt wird, als Haarkonditionierungsmittel für Zusammensetzungen in der Haarkosmetik.
Geeignete Monomere (a) sind die N-Vinylimidazol-Derivate der allgemeinen Formel (I), worin $R^1$ bis $R^3$ für Wasserstoff, $C_1$ - $C_4$ - Alkyl oder Phenyl steht.

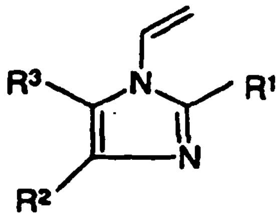

(I)

Weiterhin eignen sich Diallyamine der allgemeinen Formel (II), worin $R^4$ für $C_1$ - $C_{24}$ -Alkyl steht.

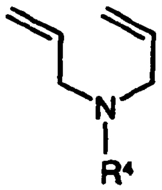

**(II)**

Zur Quaternisierung der Verbindungen der allgemeinen Formel (I) - (II) eignen sich beispielsweise Alkylhalogenide mit 1 bis 24 C-Atomen in der Alkylgruppe, z. B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternisierung der basischen Monomere der allgemeinen Formel (I) - (II) kann auch mit Alkylenoxiden wie Ethylenoxid oder Propylenoxid in Gegenwart von Säuren durchgeführt werden.

Die Quaternisierung des Monomeren oder eines Polymeren mit einem der genannten Quaternisierungsmittel kann nach allgemein bekannten Methoden erfolgen.

Bevorzugte Quaternierungsmittel sind: Methylchlorid, Dimethylsulfat oder Diethylsulfat.

Bevorzugte Monomere (a) sind 3-Methyl-1-vinylimidazoliumchlorid und -methosulfat und Dimethyldiallylammoniumchlorid, ganz besonders bevorzugt sind 3-Methyl-1-vinylimidazoliumchlorid und -methosulfat.

Als Monomere (c) eignen sich $C_1$ bis $C_{24}$, -, insbesondere $C_1$ bis $C_{10}$-Alkylester der (Meth)acrylsäure, z. B. Methyl(meth)acrylat, Ethyl(meth)acrylat, tert.-Butyl(meth)acrylat, Isobutyl(meth)acrylat, n-Butyl(meth)acrylat und Acrylamide wie N-tert.-Butylacrylamid oder N-tert.-Octylacrylamid. Ferner eignen sich Carbonsäurevinylester, z. B. Vinylacetat oder Vinylpropionat.

Monomere (d), die eine vernetzende Funktion besitzen, sind Verbindungen mit mindestens 2 ethylenisch ungesättigten, nichtkonjugierten Doppelbindungen im Molekül.

Geeignete Vernetzer sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl--1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thiopentan-1,5-diol, sowie Polyethylenglykole, Polypropylenglykole und Polytetrahydrofurane mit Molekulargewichten von jeweils 200 bis 10 000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C3- bis C6-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Monomere (d) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 - 20 000.

Als Vernetzer sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z. B. Triallylmethylammoniumchlorid oder -methylsulfat, als Vernetzer geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenharnstoff oder N,N'-Divinylpropylenharnstoff.

Weitere geeignete Vernetzer sind Divinyldioxan, Tetraallylsilan oder Tetravinylsilan.

Vorzugsweise werden solche Vernetzer eingesetzt, die in der Monomermischung löslich sind.

Besonders bevorzugt eingesetzte Vernetzer sind beispielsweise Methylenbisacrylamid, Triallylamin und Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer sind Methylenbisacrylamid, N,N'-Divinylethylenharnstoff und Acrylsäureester von Glykol, Butandiol, Trimethylolpropan oder Glycerin oder Acrylsäureester von mit Ethylenoxid und/oder Epichlorhydrin umgesetzten Glykol, Butandiol, Trimethylolpropan oder Glycerin.

Die Monomere (a) bis (d) können jeweils einzeln oder im Gemisch mit anderen Monomeren der gleichen Gruppe eingesetzt werden.

Die Herstellung der Polymerisate kann nach den an sich bekannten Verfahren der radikalisch initiierten Polymerisation, z. B. durch Lösungspolymerisation, Emulsionspolymerisation, Suspensionspolymerisation, Fällungspolymerisation, Umgekehrte Suspensionspolymerisation oder Umgekehrte Emulsionspolymerisation erfolgen, ohne daß die verwendbaren Methoden darauf beschränkt sind.

Die Polymerisation erfolgt üblicherweise bei Temperaturen von 20 °C bis 130 °C und bei Normaldruck oder unter Eigendruck.

Als Initiatoren für die radikalische Polymerisation können die hierfür üblichen wasserlöslichen und wasserunlöslichen Peroxo- und/oder Azo-Verbindungen eingesetzt werden, beispielsweise Alkali- oder Ammoniumperoxidisulfate, Dibenzoylperoxid, tert.-Butylperpivalat, tert.-Butyl-per-2-ethylhexanoat, Di-tert.-butylperoxi.d, tert.-Butylhydroperoxid, Azo-bis-isobutyronitril, Azobis-(2-amidinopropan)dihydrochlorid oder 2,2'-Azo-bis-(2-methylbutyronitril). Geeignet sind auch Initiatormischungen oder Redox-Initiator Systeme, wie z. B. Ascorbinsäure/Eisen(II)sulfat /Natriumperoxodisulfat, tert.-Butylhydroperoxid /Natriumdisulfit, tert.-Butylhydroperoxid/ Natriumhydroxymethansulfinat. Die Initiatoren können in den üblichen Mengen eingesetzt werden, beispielsweise 0,05 bis 5 Gew.-%, bezogen auf die Menge der zu polymerisierenden Monomeren.

Das Molekulargewicht und der K-Wert der Polymerisate läßt sich in an sich bekannter Weise durch die Wahl der Polymerisationsbedingungen, beispielsweise Polymerisationsdauer, Polymerisationstemperatur oder Initiatorkonzentration, und durch den Gehalt an Vernetzer in einem breiten Bereich variieren. Die K-Werte der Polymerisate liegen in einem Bereich zwischen 30 bis 350, vorzugsweise 50 bis 350.

Die K-Werte werden nach Fikentscher, Cellulosechemie, Bd. 13, S. 58-64 (1932) bei 25 °C 0,1 %ig in 0,5 molarer Kochsalzlösung gemessen.

Die erfindungsgemäßen Polymere eignen sich als Konditionierungsmittel in kosmetischen Zubereitungen,vor allem haarkosmetische Zubereitungen wie Haarkuren, Haarlotionen, Haarspülungen, Haaremulsionen, Spitzenfluids, Egalisierungsmitteln für Dauerwellen, 'Hot-Oil-Treatment'-Präparate, Conditoner, Festigerlotionen oder Haarsprays.

Je nach Anwendungsgebiet können die haarkosmetischen Zubereitungen als Spray, Schaum, Gel, Gelspray oder Mousse appliziert werden.

Die haarkosmetischen Zubereitungen können neben den erfindungsgemäßen Polymeren und geeigneten Lösungsmitteln wie Wasser oder Wasser/Alkohol-Gemischen noch in der Kosmetik übliche Zusätze wie Emulgatoren, Konservierungsmittel, Parfümöle, Pflegestoffe wie Panthenol, Collagen, Vitamine, Eiweißhydrolysate, Stabilisatoren, pH-Wert-Regulatoren, Farbstoffe, Salze, Konsistenzgeber, Silikone, Feuchthaltemittel, Rückfetter und weitere übliche Additve enthalten.

Man kann die erfindungsgemäßen Polymere auch mit herkömmlichen Haarkosmetik-Polymeren abmischen, falls ganz spezielle Eigenschaften eingestellt werden sollen.

Als herkömmliche Haarkosmetik-Polymere eignen sich beispielsweise anionische Polymere. Solche anionischen Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus t-Butylacrylat, Ethylacrylat, Methacrylsäure (z.B. Luvimer® 100P), Copolymere aus Ethylacrylat und Methacrylsäure (z.B. Luvimer® MAE), Copolymere aus N-tert.Butylacrylamid, Ethylacrylat, Acrylsäure (Ultrahold® 8, strong), Copolymere aus Vinylacetat, Crotonsäure, Vinylpropiat (z.B. Luviset® CAP), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol® VBM).

Ganz besonders bevorzugt werden als anionische Polymere Acrylate mit einer Säurezahl größer gleich 120 und Copolymere aus t-Butylacrylat, Ethylacrylat, Methacrylsäure.

Weitere geeignete Haarkosmetik-Polymere sind kationische Polymere mit der Bezeichnung Polyquaternium nach INCI, z. B. Copolymere aus Vinylpyrrolidon/ N-Vinylimidazoliumsalzen (Luviquat® FC, Luviquat® HM, Luviquat® MS), Copolymere aus N-Vinypyrrolidon /Dimethylaminoethylmethacrylat, quaternisiert mit Diethylsulfat (Luviquat® PQ 11), Copolymere aus N-Vinylcaprolactam / N-Vinylpyrrolidon / N-Vinylimidazoliumsalzen (Luviquat® Hold); kationische Cellulosederivate (Polyquaternium-4 und -10), Acrylamidcopolymere (Polyquaternium-7).

Als weitere Haarkosmetik-Polymere sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane oder Silikonharze.

Die erfindungsgemäßen Polymerisate eignen sich insbesondere zur Verwendung als Konditioniermittel in Shampoo-Formulierungen.

Die Shampooformulierungen enthalten üblicherweise anionische Tenside als Basistenside und amphotere und nichtionische Tenside als Cotenside.

Die Formulierungen enthalten 2 - 50 Gew.-% Tenside, bevorzugt 5 - 40 Gew.-%, besonders bevorzugt 8 - 30 Gew-%.

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxideinheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt weden.

Als nichtionische Tenside sind beispielsweise geeignet die Umsetzungsprodukte von aliphatischen Alkoholen oder Alkylphenolen mit 6 bis 20 C-Atomen in der Alkykette, die linear oder verzweigt sein kann, mit Ethylenoxid und/ oder Propylenoxid. Die Menge Alkylenoxid beträgt ca. 6 - 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

Die erfindungsgemäßen Polymere werden üblicherweise zwischen 0,01 - 5 Gew.-% eingesetzt, bevorzugt zwischen 0,05 und 2 Gew.-%.

Zusätzlich können auch weitere in Shampoos übliche kationische Polymere eingesetzt werden, so z. B. Copolymere aus Acrylamid und Dimethyldiallylammoniumchlorid (Polyquaternium-7), kationische Cellulosederivate (Polyquaternium-10), Guar-hydroxypropyltrimethylammoniumchlorid (INCI: Hydroxypropyl Guar Hydroxypropyltrimonium Chloride), Copolymere aus N-Vinylpyrrolidon und quaternisiertem N-Vinylimidazol (Polyquaterinium-16, -44, -46) und andere.

Weiterhin können die Shampooformulierungen Verdicker, wie z. B. Kochsalz, PEG-55, Propylene Glycol Oleate, PEG-120 Methyl Glucose Dioleate und andere, sowie Konservierungsmittel, weitere Wirkund Hilfsstoffe und Wasser enthalten.

A Herstellung der Polymere

Beispiel 1

In einer Rührapparatur wurden 400 g Wasser und 46 g Dimethyldiallylammoniumchlorid -Lösung (65 %ig) vorgelegt. Zu dieser Vorlage wurde 10 % von Zulauf 1, bestehend aus 270 g N-Vinylpyrrolidon und 0,6 g N,N'-Divinylethylenharnstoff, gegeben. Unter Rühren im Stickstoffstrom wurde auf 60 °C aufgeheizt und Zulauf 1 während 3 Stunden sowie Zulauf 2, bestehend aus 0,9 g 2,2'-Azobis(2-amidinopropan)dihydrochlorid in 100 g Wasser, während 4 Stunden zudosiert. Nach 3 Stunden verdünnte man mit 700 g Wasser und ließ eine weitere Stunde rühren. Danach gab man 1,5 g 2,2'-Azo-bis(2-amidinopropan)dihydrochlorid in 30 g Wasser zu und ließ weitere 2 Stunden bei 60 °C rühren. Man erhielt eine farblose hochviskose Polymerlösung mit einem Feststoffgehalt von 20,9 % und einem K-Wert von 80,3.

Beispiel 2

In einer Rührapparatur wurden 300 g von Zulauf 1, bestehend aus 200 g N-Vinylpyrrolidon, 77 g Dimethyldiallyammoniumchlorid-Lösung (65 %ig), 1,13 g N,N'-Divinylethylenharnstoff und 440 g Wasser, vorgelegt und unter Rühren im Stickstoffstrom auf 60 °C aufgeheizt. Der Rest von Zulauf 1 wurde in 2 Stunden und Zulauf 2, bestehend aus 0,75g 2,2'-Azo-bis(2-amidinopropan)dihydrochlorid in 100 g Wasser, in 4 Stunden zudosiert. Nach Ende von Zulauf 1 wurde das Reaktionsgemisch mit 1620 g Waser verdünnt. Nach Ende von Zulauf 2 ließ man eine weitere Stunde bei 60 °Crühren, gab danach 1,25 g 2,2'-Azo-bis(2-amidinopropan)dihydrochlorid in 65 g Wasser zu und rührte eine weitere Stunde. Man erhielt eine farblose hochviskose Polymerlösung mit einem Feststoffgehalt von 10,2 % und einem K-Wert von 80.

Beispiel 3

In einer Rührapparatur wurden 130 g Wasser und 48 g 3-Methyl-1-vinylimidazoliumchlorid vorgelegt und unter Rühren im Stickstoffstrom auf 60 °C aufgeheizt. Danach wurden Zulauf 1, bestehend aus 192 g N-Vinylpyrrolidon, 0,48 g N,N'-Divinylethylenharnstoff und 450 g Wasser, in 3 Stunden und Zulauf 2, bestehend aus 1,44 g 2,2'-Azo-bis(2-amidinopropan)dihydrochlorid in 80 g Wasser, während 4 Stunden zudosiert. Anschließend wurde noch eine Stunde bei 60 °C gerührt. Um den Ansatz rührfähig zu halten, wurde nach Bedarf mit insgesamt 2100 g Wasser verdünnt. Man erhielt eine farblose hochviskose Polymerlösung mit einem Feststoffgehalt von 8,2 % und einem K-Wert von 105.

Beispiel 4

In einer Rührapparatur wurden 716 g Wasser vorgelegt und unter Rühren im Stickstoffstrom auf 60 °C aufgeheizt. Danach wurde Zulauf 1, bestehend aus 180 g N-Vinylpyrrolidon, 20 g 3-Methyl-1-vinylimidazoliummethylsulfat, 0,32 g N,N'-Divinylethylenharnstoff und 25 g Wasser, in 2 Stunden und Zulauf 2, bestehend aus 0,6 g 2,2'-Azo-bis(2-amidinopropan)dihydrochlorid in 60 g Wasser, in 3 Stunden zudosiert. Nach Ende von Zulauf 1 wurde das Reaktionsgemisch mit 1000 g Wasser verdünnt. Im Anschluß an Zulauf 2 wurde noch 3 Stunden bei 70 °C gerührt. Man erhielt eine farblose hochviskose Polymerlösung mit einem Feststoffgehalt von 11,0 % und einem K-Wert von 86.

Beispiel 5

In einer Rührapparatur wurden 440 g Wasser vorgelegt und unter Rühren im Stickstoffstrom auf 60 °C aufgeheizt. Danach wurde Zulauf 1, bestehend aus 180 g N-Vinylpyrrolidon, 20 g 3-Methyl-1-vinylimidazoliummethylsulfat, 0,30 g N,N'-Divinylethylenharnstoff und 25 g Wasser, in 2 Stunden und Zulauf 2, bestehend aus 0,6 g 2,2'-Azo-bis(2-amidinopropan)dihydrochlorid in 60 g Wasser, in 3 Stunden zudosiert. Im Anschluß an Zulauf 2 wurde noch 3 Stunden bei 70 °C gerührt. Um das Reaktionsgemisch rührfähig zu halten, wurde bei Bedarf insgesamt mit 1275 g Wasser verdünnt. Man erhielt eine farblose hochviskose Polymerlösung mit einem Feststoffgehalt von 11,3 % und einem K-Wert von 105.

Beispiel 6

In einer Rührapparatur wurden 650 g Wasser vorgelegt und unter Rühren im Stickstoffstrom auf 60 °C aufgeheizt. Danach wurde Zulauf 1, bestehend aus 225 g N-Vinylpyrrolidon, 25 g 2,3-Dimethyl-1-vinylimidazoliummethylsulfat, 0,25 g N,N'-Divinylethylenharnstoff und 580 g Wasser, in 3 Stunden und Zulauf 2, bestehend aus 0,7 g 2,2'-Azo-bis(2-amidinopropan)dihydrochlorid in 100 g Wasser, in 4 Stunden zudosiert. Nach Ende von Zulauf 1 wurde das Reaktionsgemisch mit 835 g Wasser verdünnt. Im Anschluß an Zulauf 2 wurde noch eine Stunde gerührt und 1,25 g 2,2'-Azobis(2-amidinopropan)dihydrochlorid in 77 g Wasser nachdosiert. Danach rührte man noch 2 Stunden bei 70 °C. Man erhielt eine farblose hochviskose Polymerlösung mit einem Feststoffgehalt von 10,4 % und einem K-Wert von 106.

Beispiel 7

In einer Rührapparatur wurden 650 g Wasser vorgelegt und unter Rühren im Stickstoffstrom auf 60 °C aufgeheizt. Danach wurde Zulauf 1, bestehend aus 225 g N-Vinylpyrrolidon, 25 g 2,3-Dimethyl-1-vinylimidazoliummethylsulfat, 0,375 g N,N'-Divinylethylenharnstoff und 580 g Wasser, in 3 Stunden und Zulauf 2, bestehend aus 0,7 g 2,2'-Azo-bis (2-amidinopropan)dihydrochlorid in 100 g Wasser, während 4 Stunden zudosiert. Nach Ende von Zulauf 1 wurde das Reaktionsgemisch mit 1135 g Wasser verdünnt. Im Anschluß an Zulauf 2 wurde noch eine Stunde gerührt und 1,25 g 2,2'-Azo-bis(2-amidinopropan)dihydrochlorid in 77 g Wasser nachdosiert. Danach rührte man noch 2 Stunden bei 70 °C. Man erhielt eine farblose hochviskose Polymerlösung mit einem Feststoffgehalt von 9,2 % und einem K-Wert von 92.

Beispiel 8

In einem Reaktionsgefäß mit Stickstoffspülung wurden 800 g Cyclohexan, 5 g Sorbitanmonooleat, 5 g Hypermer B246[1] und 1 g 2,2'-Azobis(2,4-dimethylvaleronitril) vorgelegt und auf 65 °C erwärmt. Der [1] Hypermer B24® polymeres Tensid der Fa. ICI Zulauf, bestehend aus 100 g 3-Methyl-1-vinylimidazoliummethylsulfat, 100 g N-Vinylpyrrolidon, 100 g Wasser und 0,25 g Tripropylenglycoldiacrylat, wurde innerhalb 20 Minuten zudosiert. Anschließend wurde 6 Stunden bei 65 °C gerührt. Dann wurden 200 g Cyclohexan zugesetzt und das Wasser azeotrop abdestilliert, das Polymerisat abfiltriert und getrocknet. Der K-Wert einer wässerigen Lösung des Polymerisates betrug 114.

Beispiel 9

In einer Rührapparatur wurden 900 g Ethylacetat vorgelegt und unter Rühren im Stickstoffstrom auf 77 °C aufgeheizt. Danach wurde Zulauf 1, bestehend aus 270 g N-Vinylpyrrolidon, 30 g 1-Vinylimidazol und 0,3 g N,N'-Divinylethylenharnstoff, in 3 Stunden und Zulauf 2, bestehend aus 3 g 2,2'-Azo-bis(2-methylbutyronitril) in 80 g Ethylacetat, während 4 Stunden zudosiert. Danach rührte man noch 2 Stunden, kühlte auf Raumtemperatur ab und versetzte mit 36 g Dimethylsulfat. Anschließend rührte man eine halbe Stunde bei Raumtemperatur und weitere 2 Stunden bei 70 °C. Das erhaltene Pulver wurde abfiltriert und getrocknet. Der K-Wert einer wässerigen Lösung des Polymerisates betrug 125.

Beispiel 10:

In einer Rührapparatur wurden 440 g Wasser vorgelegt und unter Rühren im Stickstoffstrom auf 60 °C aufgeheizt. Danach wurde Zulauf 1, bestehend aus 144 g N-Vinylpyrrolidon, 16 g 3-Methyl-1-vinylimidazoliummethylsulfat, 1,4 g Tetraethylenglykoldiacrylat und 100 g Wasser, in 2 Stunden und Zulauf 2, bestehend aus 0,8 g 2,2'-Azo-bis(2-amidinopropan)dihydrochlorid in 50 g Wasser, in 3 Stunden zudosiert. Im Anschluß an Zulauf 2 wurde noch 3 Stunden bei 70 °C gerührt. Um das Reaktionsgemisch rührfähig zu halten, wurde bei Bedarf insgesamt mit 1200 g Wasser verdünnt. Man erhielt eine farblose hochviskose Polymerlösung mit einem Feststoffgehalt von 8,5 % und einem K-Wert von 95.

Beispiel 11:

In einer Rührapparatur wurden 550 g Wasser vorgelegt und unter Rühren im Stickstoffstrom auf 60 °C aufgeheizt. Danach wurde Zulauf 1, bestehend aus 102 g N-Vinylpyrrolidon, 26 g 3-Methyl-1-vinylimidazoliummethylsulfat, 0,8 g Triallylamin und 100 g Wasser in 2 Stunden zudosiert. Zulauf 2, bestehend aus 0,6 g 2,2'-Azo-bis(2-amidinopropan) dihydrochlorid in 50 g Wasser, wurde in 3 Stunden zur Reaktionsmischung zugegeben. Im Anschluß an Zulauf 2 wurde noch 3 Stunden bei 70 °C gerührt. Um das Reaktionsgemisch rührfähig zu halten, wurde bei Bedarf insgesamt mit 1000 g Wasser verdünnt. Man erhielt eine schwach gelbliche, hochviskose Polymerlösung mit einem Feststoffgehalt von 7,0 % und einem K-Wert von 102.

Beispiel 12:

Beispiel 11 wurde wiederholt, nur wurde anstelle von Triallylamin 2,2 g Pentaerythrittriallylether eingesetzt. Man erhielt eine schwach gelbliche, hochviskose Polymerlösung mit einem K-Wert von 95.

Beispiel 13:

In einer Rührapparatur wurden 440 g Wasser vorgelegt und unter Rühren im Stickstoffstrom auf 60 °C aufgeheizt. Danach wurde Zulauf 1, bestehend aus 150 g N-Vinylpyrrolidon, 8 g 3-Methyl-1-vinylimidazoliummethylsulfat, 0,6 g Triallylamin und 100 g Wasser, in 2 Stunden und Zulauf 2, bestehend aus 0,8 g 2,2'-Azo-bis(2-amidinopropan)dihydrochlorid in 50 g Wasser, in 3 Stunden zudosiert. Im Anschluß an Zulauf 2 wurde noch 3 Stunden bei 70 °C gerührt. Um das Reaktionsgemisch rührfähig zu halten, wurde bei Bedarf insgesamt mit 1200 g Wasser verdünnt. Man erhielt eine farblose hochviskose Polymerlösung mit einem Feststoffgehalt von 8,1 % und einem K-Wert von 98.

Beispiel 14:

In einem Reaktionsgefäß mit Stickstoffspülung wurden 800 g Cyclohexan, 5 g Sorbitanmonooleat und 5 g Hypermer B246[2] vorgelegt und auf 60 °C erwärmt. Zulauf 1, bestehend aus 60 g 3-Methyl-1-vinylimidazoliummethylsulfat, 140 g N-Vinylpyrrolidon, 150 g Wasser und 1,0 g Triallylamin, und Zulauf 2, bestehend aus 0,6 g 2,2'-Azo-bis(2-amidinopropan)dihydrochlorid in 50 g Wasser, wurden innerhalb 1 Stunde zudosiert. Anschließend wurde weitere 6 Stunden bei 60 °C gerührt. Dann wurden 200 g Cyclohexan zugesetzt und das Wasser azeotrop abdestilliert, das Polymerisat abfiltriert und getrocknet.

Beispiel 15:

In einem Reaktionsgefäß mit Stickstoffspülung wurden 800 g Cyclohexan, 5 g Sorbitanmonooleat und 5 g Hypermer B246[3] vorgelegt und auf 60 °C erwärmt. Zulauf 1, bestehend aus 20 g 3-Methyl-1-vinylimidazoliummethylsulfat, 180 g N-Vinylpyrrolidon, 150 g Wasser und 0,5 g Triallylamin, wurde innerhalb 1 Stunde und Zulauf 2,
[2] Hypermer B24® polymeres Tensid der Fa. ICI
[3] Hypermer B24® polymeres Tensid der Fa. ICI bestehend aus 1,2 g 2,2'-Azo-bis(2-amidinopropan)dihydrochlorid in 70 g Wasser, innerhalb 4 Stunden zudosiert. Anschließend wurde weitere 3 Stunden bei 60 °C gerührt. Dann wurden 200 g Cyclohexan zugesetzt und das Wasser azeotrop abdestilliert, das Polymerisat abfiltriert und getrocknet.

Beispiel 16

In einer Rührapparatur wurden 400 g Wasser, 100 g N-Vinylpyrrolidon, 11 g 3-Methyl-1-vinylimidazoliummethylsulfat und 0,4 g Triallylamin vorgelegt und unter Rühren im Stickstoffstrom auf 60°C aufgeheizt. Danach wurde Zulauf 1, bestehend aus 0,6 g 2,2'-Azobis(2-amidinopropan)dihydrochlorid in 50 g Wasser, in 3 Stunden zur Reaktionsmischung zugegeben und mit 1000 g Wasser verdünnt. Anschließend wurde noch 3 Stunden bei 80°C gerührt. Man erhielt eine farblose, hochviskose Polymerlösung mit einem Feststoffgehalt von 7,6 % und einem K-Wert von 110.

B Verwendung der Polymere als Konditionierungsmittel

Beispiele 17-22.

Im folgenden wurden 6 Shampoos gemäß untenstehender Formulierung unter Verwendung der Polymere aus den Beispielen 1-15 hergestellt und ihre haarkosmetischen Eigenschaften ermittelt.

Beispiele 23-25 (Vergleichsversuche)

Es wurden 3 Shampoos gemäß untenstehender Formulierung hergestellt, wobei jedoch kationische Polymere eingesetzt wurden, die keinen Vernetzer enthielten.
Shampooformulierung für Beispiele 17-25 in Tabelle 1:

| | |
|---|---|
| Natriumlaurylethersulfat | 10.0 % |
| Cocamidopropylbetain | 4.0 % |
| Polymer (aus Beispiel 1-15) | 0.1 bzw. 0.5 % |
| Wasser | ad 100 % |

Testmethoden:

a) Naßkämmbarkeit/Trockenkämmbarkeit

An einer Zug/Druck-Prüfmaschine wird die Kämmbarkeit bestimmt, welche notwendig ist, einen Kamm durch eine Haartresse zu ziehen. Die Kämmkraftabnahme wird wie folgt berechnet (je größer der Wert, desto besser das Shampoo).

**% Kämmkraftabnahme = 100 ( 1 - Ay/Ao)**

Ay = Kämmarbeit nach Behandlung mit Testshampoo (siehe Beispiele)
Ao = Kämmarbeit nach Behandlung mit Shampoo ohne Polymer (Blindwert)

b) Schaumcremigkeit

Subjektive Bewertung mit einer Notenskala von 1 (sehr gut) bis 3 (schwach)
Sowohl die Kämmbarkeit des Haares als auch die Schaumcremigkeit wird durch die Art und Menge des Polymers beeinflußt.

Tabelle 1:

| Anwendungstechnische Prüfungen mit dem oben genannten Testshampoo | | | | |
|---|---|---|---|---|
| Shampoo Beispiel Nr | Herstellbeispiel Nr | Kämmkraft-abnahme naß 0.1 %/ 0.5 % Polymer | Kämmkraft-abnahme trocken 0.5 % Polymer | Schaumcremigkeit (Note) |
| 17 | 1 | 47 %/ 57 % | — | sehr gut |
| 18 | 4 | 55 %/ 64 % | 48 % | sehr gut |
| 19 | 7 | 52 %/ 60 % | - | sehr gut |
| 20 | 11 | 37 % / 55 % | - | sehr gut |
| 21 | 14 | 62 % / 65 % | | sehr gut |
| 22 | 15 | 51 % / 66 % | — | sehr gut |
| 23 | Polyquaternium—16 | 0 % / 19 % | minus 40 % | schwach |
| 24 | Polyquaternium —7 | 12 %/ 24 % | 24 % | gut |
| 25 | Polyquaternium—10 | 21 %/ 29 % | 21 % | gut |

Die Beispiele 17 - 22 zeigen deutlich die hervorragenden Eigenschaften bei erfindungsgemäßer Verwendung gegenüber bekannter Verwendung (Beispiele 23 - 25).

**Patentansprüche**

**1.** Verwendung von Polymeren, die erhältlich sind durch

(i) radikalisch initiierte Copolymerisation von Monomergemischen aus

(a) 2 bis 70 Gew.-% eines kationischen Monomeren oder quatemisierbaren Monomeren,
wobei als Monomer (a)
N-Vinylimidazol-Derivate der allgemeinen Formel (I),

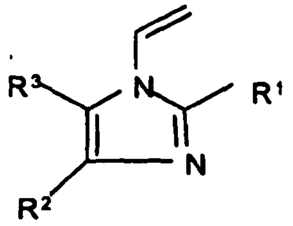

(I)

worin $R^1$ bis $R^3$ für Wasserstoff, C1 - C4 -Alkyl oder Phenyl steht, oder Diallylamine der allgemeinen Formel (II),

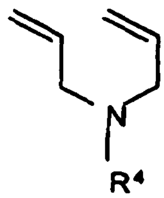

(II)

worin $R^4$ für C1 - C24 -Alkyl steht
oder
3-Methyl-1-vinylimidazoliumchlorid, -methosulfat, Dimethyldiallyammoniumchlorid
verwendet werden,
(b) 22-97,98 Gew.-% N-Vinylpyrrolidon,
(c) 0 bis 40 Gew.-% eines weiteren radikalisch copolymerisierbaren Monomeren und
(d) 0,02 bis 8 Gew.-% eines bi- oder mehrfunktionellen radikalisch copolymerisierbaren Monomeren, und

(ii) anschließende Quaternisierung des Polymeren, sofern als Monomeres (a) ein nicht quatemisiertes Monomer eingesetzt wird,

als Haarkonditionierungsmittel in Zubereitungen für die Haarkosmetik.

**2.** Verwendung nach Anspruch 1 in Shampoos.

**Claims**

**1.** The use of a polymer obtainable by

(i) free-radically initiated copolymerization of monomer mixtures comprising

(a) 2% to 70% by weight of a cationic monomer or quaternizable monomer,
use being made as monomer (a) of N-vinylimidazole derivatives of the general formula (I),

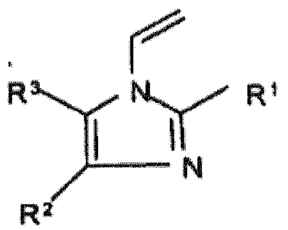

(I)

in which $R^1$ to $R^3$ are hydrogen, C1 - C4 alkyl or phenyl,
or
diallylamines of the general formula (II),

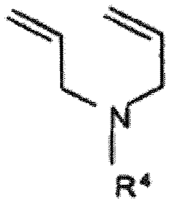

(II)

in which $R^4$ is C1 - C24 alkyl or
3-methyl-1-vinylimidazolium chloride or methosulfate, or dimethyldiallylammonium chloride,
(b) 22-97.98% by weight of N-vinylpyrrolidone,
(c) 0% to 40% by weight of a further free-radically copolymerizable monomer and
(d) 0.02% to 8% by weight of a bifunctional or polyfunctional, free-radically copolymerizable monomer, and

(ii) subsequent quaternization of the polymer if as monomer (a) a non-quaternized monomer is employed,

as a hair conditioning agent in a preparation for hair cosmetics.

**2.** The use according to claim 1 in a shampoo.

**Revendications**

**1.** Utilisation de polymères qui peuvent être obtenus par

(i) copolymérisation à amorçage radicalaire de mélanges de monomères composés de

(a) 2 à 70 % en poids d'un monomère cationique ou monomère apte à la quaternisation,
en tant que monomère (a) étant utilisés des dérivés de N-vinylimidazole de formule générale (I),

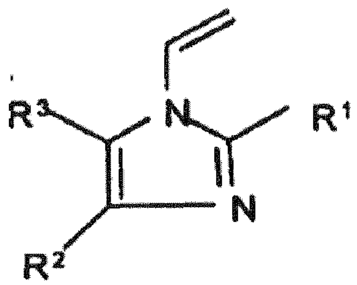

(I)

dans laquelle $R^1$ à $R^3$ représentent un atome d'hydrogène, un groupe alkyle en $C_1$-$C_4$ ou phényle, ou des diallylamines de formule générale (II),

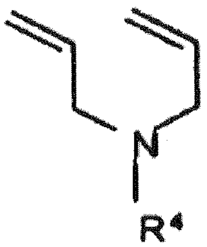

(II)

dans laquelle $R^4$ représente un groupe alkyle en $C_1$-$C_{24}$

ou
du chlorure, méthosulfate de 3-méthyl-1-vinylimidazolium, du chlorure de diméthyldiallylammonium,
(b) 22-97,98 % en poids de N-vinylpyrrolidone,
(c) 0 à 40 % en poids d'un autre monomère copolymérisable par voie radicalaire et
(d) 0,02 à 8 % en poids d'un monomère bi- ou polyfonctionnel, copolymérisable par voie radicalaire, et

(ii) quaternisation subséquente du polymère, lorsqu'on utilise comme monomère (a) un monomère non rendu quaternaire,

en tant que conditionneur pour cheveux dans des préparations pour la cosmétique capillaire.

**2.** Utilisation selon la revendication 1 dans des shampooings.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 246580 A
- EP 544158 A
- US 4859756 A
- EP 715843 A
- DE 3209224
- US 4058491 A
- WO 9626229 A
- WO 9637525 A
- DE 4213971
- DE 2821239
- US 4348380 A
- DE 3106974
- US 5275809 A
- EP 522755 A
- EP 521665 A
- EP 521666 A
- US 4806345 A
- WO 9325595 A
- EP 0671157 A